# EUROPEAN PATENT APPLICATION

(11) **EP 1 452 595 A1**
(43) Date of publication of application: **01.09.2004**
(21) Application number: 02783558.6
(22) Date of filing: 13.11.2002
(51) Int. Cl.: C12N 15/09, C12N 5/10, A01K 67/027

(54) **ES CELLS WITH ENHANCED RNAi EFFECT**

(30) Priority: 14.11.2001 JP 2001348705
(71) Applicant: Gencom Corporation, Machida-shi, Tokyo 194-8511 (JP)
(72) Inventor: KATSUKI, Motoya, Minato-ku, Tokyo 106-0032 (JP); ISHIDA, Mitsuyoshi, c/o GENCOM CORPORATION, Machida-shi, Tokyo 194-8511 (JP); KATO, Minoru, c/o GENCOM CORPORATION, Machida-shi, Tokyo 194-8511 (JP)
(74) Representative: Sternagel, Fleischer, Godemeyer & Partner Patentanwälte
(86) International application number: PCT/JP2002/011831
(87) International publication number: WO 2003/042382

(57) **Abstract**

The object of the present invention is to provide ES cells and mammals having enhanced RNAi effect, which can be used to analyze gene functions at an individual level. The present invention provides ES cells having enhanced RNAi effect, which are obtained by performing genetic manipulation on ES cells.

SEQUENCE LISTING
<110> GenCom
<120> ES cell having an enhanced RNAi effect
<130> A21594A
<160> 17

<210> 1
<211> 35
<212> DNA
<213> Artificial Sequence
<220>
<223> Description of Artificial Sequence: Synthetic DNA
<400> 1
ggccatatag gccccgaccc gcagtcccag cgtcg 35

<210> 2
<211> 38
<212> DNA
<213> Artificial Sequence
<220>
<223> Description of Artificial Sequence: Synthetic DNA
<400> 2
ggcctacatg gccttaggct ttgtatttgg cttttcca 38

<210> 3
<211> 32
<212> DNA

<213> Artificial Sequence

<220>

<223> Description of Artificial Sequence: Synthetic DNA

<400> 3

cggtccgatg ccgacccgca gtcccagcgt cg 32

<210> 4

<211> 32

<212> DNA

<213> Artificial Sequence

<220>

<223> Description of Artificial Sequence: Synthetic DNA

<400> 4

cggaccgtta ggctttgtat ttggcttttc ca 32

<210> 5

<211> 20

<212> DNA

<213> Artificial Sequence

<220>

<223> Description of Artificial Sequence: Synthetic DNA

<400> 5

aattgactag tgcgcgcatg 20

<210> 6

<211> 20

<212> DNA

<213> Artificial Sequence

<220>

<223> Description of Artificial Sequence: Synthetic DNA

<400> 6

aattcatgcg cgcactagtc                                        20

<210> 7

<211> 20

<212> DNA

<213> Artificial Sequence

<220>

<223> Description of Artificial Sequence: Synthetic DNA

<400> 7

attctagatc gtcgacattt                                        20

<210> 8

<211> 20

<212> DNA

<213> Artificial Sequence

<220>

<223> Description of Artificial Sequence: Synthetic DNA

<400> 8

aaatgtcgac gatctagaat                                        20

<210> 9

<211> 20

<212> DNA

<213> Artificial Sequence

<220>

<223> Description of Artificial Sequence: Synthetic DNA

<400> 9

ccgggaagtt ctgaaagcaa                                        20

<210> 10

<211> 20

<212> DNA

<213> Artificial Sequence

<220>

<223> Description of Artificial Sequence: Synthetic DNA

<400> 10

tagccatatc tgcagcaacg                                        20

<210> 11

<211> 12

<212> DNA

<213> Artificial Sequence

<220>

<223> Description of Artificial Sequence: Synthetic DNA

<400> 11

agctcagatc tg                                                12

<210> 12

<211> 35

<212> DNA

<213> Artificial Sequence

<220>

<223> Description of Artificial Sequence: Synthetic DNA

<400> 12

cgcgcagcgg ccgcgatatc agctagcagg tacca          35

<210> 13

<211> 35

<212> DNA

<213> Artificial Sequence

<220>

<223> Description of Artificial Sequence: Synthetic DNA

<400> 13

cgcgtggtac ctgctagctg atatcgcggc cgctg          35

<210> 14

<211> 22

<212> DNA

<213> Artificial Sequence

<220>

<223> Description of Artificial Sequence: Synthetic DNA

<400> 14

aaatggcgcg ccttaattaa gc                        22

<210> 15

<211> 22

<212> DNA

<213> Artificial Sequence

<220>

<223> Description of Artificial Sequence: Synthetic DNA

<400> 15

gcttaattaa ggcgcgccat tt                                22

<210> 16

<211> 22

<212> DNA

<213> Artificial Sequence

<220>

<223> Description of Artificial Sequence: Synthetic DNA

<400> 16

tggcgcgcct taattaactg ca                                22

<210> 17

<211> 22

<212> DNA

<213> Artificial Sequence

<220>

<223> Description of Artificial Sequence: Synthetic DNA

<400> 17

gttaattaag gcgcgccatg ca                                22

## Description

### Technical Field

The present invention relates to ES cells having enhanced RNAi effect, and more specifically, ES cells having enhanced RNAi effect which are obtained by performing genetic manipulation on ES cells.

### Background Art

In order to clarify the functions of DNA at an individual level, a method comprising producing a gene knockout animal and analyzing its phenotype has been applied so far. However, this knockout method requires enormous manpower and time, and thus, it is not practical for analyzing the functions of a large number of genes. Accordingly, it is desired to develop a method for repressing the functions of genes in an individual animal, which is more effective and simple than conventional knockout methods.

RNAi (RNA interference) is a phenomenon wherein, when RNA (double stranded RNA: dsRNA) obtained by converting a part of mRNA encoding a part of a certain gene (referred to also as a target gene) into a double strand is introduced into cells, the expression of the target gene is inhibited. In 1998, it was discovered in nematodes that *in vivo* introduction of DsRNA has an action of repressing the expression of the same gene as the introduced gene (Fire et al., Nature, 391, 806-811, 1998).

Thereafter, RNAi was observed in Eumycetes, *Nicotiana tabaccum* and *Oryza sativa* plants, planaria, *Trypanosoma brucei* (Ngo, H., Tschudi, C., Gull, K., and Ullu, E.), the *Drosophila melanogaster* fly (Kennerdell and Carthew, 1998), and even zebrafish that is Vertebrata. Thus, it has been considered that RNAi is a phenomenon that is universally present, regardless of species.

With regard to the technical application of RNAi, it has been established as a gene knockout technique in nematodes, and it is used as a principal means for genome function analysis using information regarding the entire nucleotide sequence obtained as a result of the project of determining the entire nematode genome sequence (Fraser et al., Nature, 408, 325-330, 2000; Gonczy et al., Nature 408, 331-336, 2000). Also in the genome function analysis of mammals, the RNAi technique is expected as a method requiring less of time and manpower burden than the gene knockout method, and as a method for efficiently repressing the expression of genes.

With regard to mammals, RNAi effect has been reported for the first time in an experiment wherein dsRNA was injected into the early embryo of a mouse (Wianny and Zernikca-Goetz, Nature Cell Biol., 2, 70-75, 2000). However, this RNAi effect was observed only in the early embryo, and at the time when the mouse was born, the effect had disappeared. The reason is considered to be the fact that the dsRNA introduced in a one-cell stage fertilized ovum is diluted during the segmentation and growth of the embryo, and that a concentration thereof necessary for the RNAi cannot be maintained. In addition, there is another possibility that mammals might have a biologically different mechanism from nematodes.

For the purpose of maintaining the intracellular concentration of the introduced dsRNA, the present inventors have constructed a gene by ligating a gene comprising an inverted repeat sequence downstream of a mammalian expression vector, introduced the gene into a fertilized ovum, and then implanted the obtained embryo in the oviduct of a mouse, so as to produce a mouse into which a dsRNA expression vector gene was introduced. In the thus produced mice, it was observed that several mice showed the phenotype of repressed expression of a target gene. However, the efficiency of success is several percents, and thus, further improvement is desired to carry out the gene function analysis of individual mice, utilizing the RNAi effect.

As an attempt to identify RNAi-associated genes, mutant nematodes deficient in sensitivity to RNAi have been selected, whereby 4 RNAi-associated gene loci, rde-1 to rde-4 (rde: RNA interference-deficient), have been identified. The rde-1 gene encodes a protein (RDE-1) consisting of 1,020 amino acids. The nematode genome contains at least 22 genes having a nucleotide sequence homologous to the rde-1 gene, and these genes form a gene family. The genes show homology also with the zwille (=pinhead) and argonautel of Arabidopsis, the sting and piwi of drosophila, and the eIF2C gene of rabbits.

As another method of identifying RNAi-associated genes, RNAi sensitivity to existing mutant nematode strains was examined. As a result, it was found that the activity of RNAi was significantly reduced mainly in the germ lines in 2 mutator strains, mut-2 and mut-7 (which have a mutation causing a high frequency of transposon transfer). The sequence of the mut-7 product shows homology with RNaseD of bacteria or the 3', 5'-nuclease domain of a human Werner-syndrome protein. As a result of several experiments, the possibility is suggested that degradation of mRNA might occur in RNAi through the catalytic action of a complex comprising many proteins such as mut-7 product.

In addition, it is also suggested in nematode that a causative gene of an ego-1 mutant resulting in abnormal oogenesis plays an important role also in RNAi. An EGO-1 protein has a sequence homologous to those of tomato RNA-directed RNA polymerase (RdRP) or of QDE-1 exhibiting a gene quelling function in *Neurospora crassa.*

Moreover, Hannon et al., have reported that one nuclease belonging to drosophila RNase III family has an activity of cleaving dsRNA into 22 nucleotide RNA fragments, and that this nuclease is required for the activity of RNAi. This nuclease named "Dicer" has two RNase III motifs, and a helicase domain at the N terminus.

Dicer has the RNase III motifs as well as a region known as a PAZ domain. The PAZ domain is commonly contained in several factors (Piwi, Argo, and Zwille/Pinhead), which are predicted to be involved in RNAi. Its function has not been clarified yet, but it is considered that it functions for protein-protein interactions.

As stated above, there have been reports on factors which are involved in RNAi, but there have been no report that RNAi effect is enhance in vivo by practically using such factors.

### Disclosure of the Invention

It is an object of the present invention to provide ES cells and mammals having enhanced RNAi effect, which can be used to analyze gene functions at an individual level.

The present inventors have conducted intensive studies to achieve the above object. In order to obtain higher RNAi effect, they have studied the RNAi effect using cultured cells (ES cells). They have first confirmed the RNAi effect in ES cells, and have then attempted to achieve high sensitivity of RNAi effect.

More specifically, first, various genes involved in RNAi were ligated downstream of a part of the chicken β-globin gene insulator sequence (240 base pairs), the CMV enhancer, and the human EF1α promoter. Thereafter, a SV40 poly (A) addition signal was added, and a construct of puromycin-resistant gene, which was sandwiched between loxp sequences, was also added. Thereafter, this gene was introduced into an EGFP-expressing ES cell line. Using this cell line, EGFP fluorescence was analyzed during the transient expression of an EGFP dsRNA-expressing gene. As a result, it was found that when compared with a control ES cell line in which no RNAi-associated genes were introduced, the ratio of cell groups with reduced EGFP fluorescence was increased. This is to say, the present inventors have succeeded in obtaining an ES cell line that is highly sensitive to RNAi by introducing an RNAi-associated gene, thereby completing the present invention.

Moreover, accordingly to conventional methods, an ES cell line highly sensitive to RNAi was introduced into a mouse blastcyst embryo, and the embryo was then implanted in a uterus, so as to produce a transgenic mouse. This transgenic mouse was considered to be highly sensitive to RNAi, and reduction in EGFP fluorescence would be more significantly observed in the mouse. This is to say, by introducing the ES cells highly sensitive to RNAi which was produced in the present invention into the mouse blastcyst embryo, a mouse having properties of these ES cells can be produced.

Thus, the present invention provides ES cells having enhanced RNAi effect, which are obtained by performing genetic manipulation on ES cells.

In preferred embodiments of the present invention, the followings are provided:
ES cells which are obtained by introducing an RNAi-associated gene into ES cells;
ES cells wherein the RNAi-associated gene is a gene encoding a factor associated with the formation of a sequence-specific intermediate, a gene encoding a factor associated with a stage of repressing a target gene expression, a gene encoding RNA-dependent RNA polymerase, or a gene encoding helicase;
ES cells wherein the RNAi-associated gene is nematode rde-1 or rde-4 gene, fungus qde-2 gene, Arabidopsis ago-1 gene, Dicer gene or homologous genes thereof, a gene encoding PAZ/Piwi family proteins, nematode mut-7 gene, nematode rde-2 gene, fungus qde-1 gene, nematode ego-1 gene, Arabidopsis sgs2/sde1 gene, fungus qde-3 gene, nematode smg-2 gene, Chlamydomonas mut-6 gene, or Arabidopsis sde-3 gene;
ES cells wherein the RNAi-associated gene is nematode rde-1 gene or nematode mut-7 gene;
ES cells which are obtained by introducing into ES cells an expression vector which has an RNAi-associated gene in such a state that the gene can be expressed in host cells;
ES cells which further comprises a recombinant gene comprising an inverted repeat sequence of a target gene that can be expressed in mammalian cells;
ES cells wherein the recombinant gene comprising an inverted repeat sequence comprises the inverted repeat sequence of the target gene downstream of a promoter sequence capable of operating in mammalian cells;
ES cells wherein the recombinant gene comprising an inverted repeat sequence comprises an enhancer sequence upstream of the promoter sequence;
ES cells wherein the recombinant gene comprising an inverted repeat sequence further comprises an insulator sequence or a part thereof;
ES cells wherein the recombinant gene comprising an inverted repeat sequence comprises a poly(A) addition signal sequence downstream of the inverted repeat sequence of the target gene;
ES cells wherein the target gene is a gene of a foreign reporter protein or a mutant protein thereof;
ES cells wherein the foreign reporter protein is an enhanced green fluorescent protein (EGFP); and
ES cells, which have Accession No. FERM BP-8208 (transferred from FERM P-18574) or FERM BP-8209 (transferred from FERM P-18575).

In another aspect of the present invention, there is provided a non-human mammal derived from the above-described ES cells of the present invention, or progenies thereof, or a part thereof.

The non-human mammal is preferably one selected from the group consisting of mouse, rat, hamster, guinea pig, rabbit, canine, feline, horse, bovine, sheep, swine, goat, and monkey.

### Brief Description of the Drawings

Figure 1 shows the construction of pCE HPRT IR construct.
Figure 2 shows the structure of pUC19 5' INS240 CE pA vector.
Figure 3 shows the structure of a pBS/loxP/PURO vector.

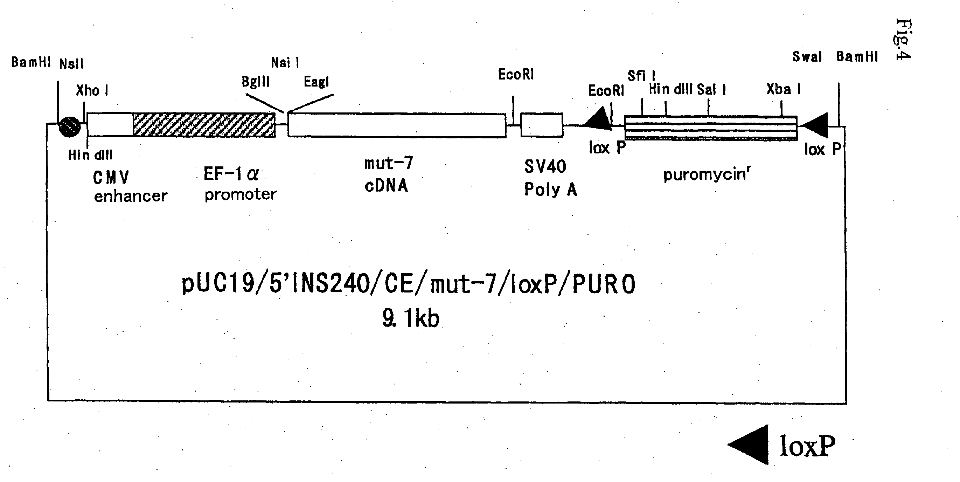

Figure 4 shows the structure of pUC19/5' INS240/CE/mut-7/loxP/PURO vector.
Figure 5 shows the structure of pUC19/5' INS240/CE/rde-1/loxP/PURO vector.
Figure 6 shows the RNAi effects (1) of ES cell line into which a gene has been introduced.

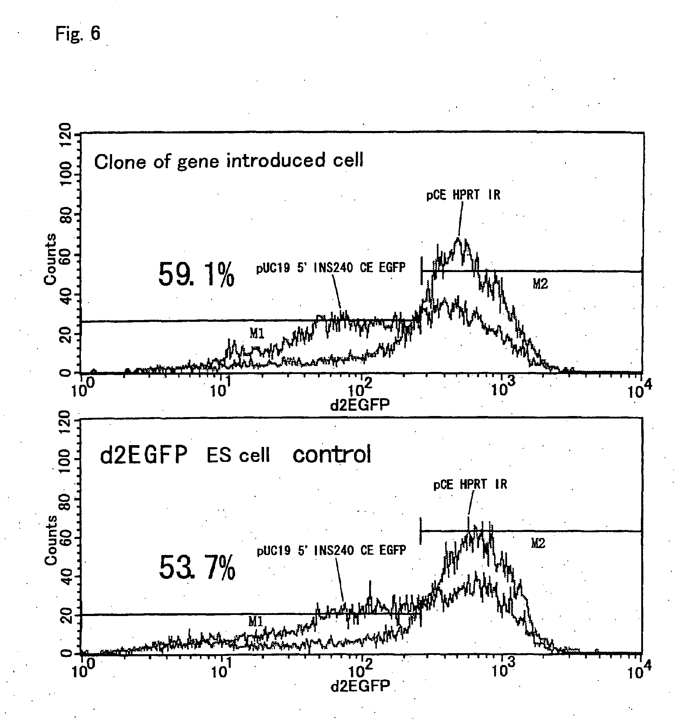

The rde-1-expressing gene was introduced into d2EGFP-expressing ES cell line, d2GFP32. The thus obtained cell line was transfected with pUC19 5' INS240 CE EGFP (black) or pCE HPRT IR (grey). 24 hours later, the cells were recovered and then were subjected to flow cytometry analysis. Figure 6 shows the results of this analysis.

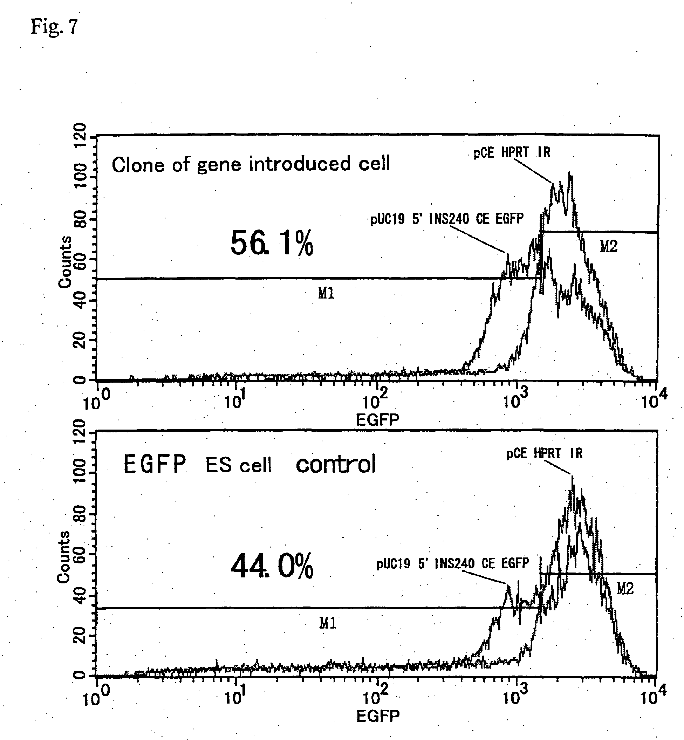

Figure 7 shows the RNAi effects (2) of a gene-introduced ES cell line.

The rde-1-expressing gene was introduced into EGFP-expressing ES cell line, GFP11. The thus obtained cell line was transfected with pUC19 5' INS240 CE EGFP (black) or pCE HPRT IR (grey). 24 hours later, the cells were recovered and then were subjected to flow cytometry analysis. Figure 7 shows the results of this analysis.

### Best Mode for Carrying Out the Invention

The embodiments of the present invention will be described in detail below.

### (1) Concerning RNAi-associated gene

The ES cells of the present invention are characterized in that the cells have an enhanced RNAi effect, and the cells are obtained by performing genetic manipulation on ES cells.

Such genetic manipulation may include the introduction of a gene enhancing the RNAi effect (that is, RNAi-associated gene) into ES cells, and the regulation of the expression of a gene which repressing the RNAi effect which exists in ES cells.

Examples of genes that are likely to increase RNAi sensitivity, that is, examples of genes enhancing RNAi effect, are given below.

RNAi was initially found in a nematode. Thereafter, it was reported that RNAi also exists in drosophila, zebrafish, and mouse. On the other hand, it has been clarified that plants have post-transcriptional gene silencing (PTGS), a mechanism similar to that of the reported phenomenon, and fungi have gene quelling that is also a mechanism similar to that of the reported phenomenon.

The following factors are considered to be associated with the formation of a sequence-specific intermediate at the first stage of the RNAi mechanism:

Nematode rde-1 and rde-4 (rde: RNA interference-deficient) genes (Tabara, H., Sarkissian, M., Kelly, W. G., Fleenor, J., Grishok, A., Timmons, L., Fire, A., Mello, C. C. : Cell. 99, 123-132, 1999).

The rde-1 gene belongs to a piwi/sting/argonaute/Zwille/eIF2C gene family. As a fungal homolog of the rde-1 gene, a qde-2 gene has been reported (Catalanptto, C., Azzalin, G., Macino, G. and Cogoni, C., Nature 404, 245, 16 Mar. 2000). As an Arabidopsis homolog thereof, an ago-1gene has been reported.

Dicer is a nuclease belonging to the drosophila RNaseIII family, and its homologs have been found in nematodes, Arabidopsis, humans, fission yeast, and the like (Bernstein, E., Caudy, A. A., Hamond S. M., Hannon, G. J.: Nature, 409, 363-366, 2001).

A protein belonging to the PAZ/Piwi family that interacts with Dicer (Hammond, S. M., Boettcher, D., Caudy, A., Kobayashi, R, Hannon, G. J., Science, 2001, 293, 1146-1150) is an example of another component of a sequence-specific intermediate.

On the other hand, the following factors are considered to be associated with a stage of repressing the target gene expression.

The nematode mut-7 (Ketting, R F., Harverkamp, T. H. A., Van Luenen, H. G. A. M., Plasterk, R H. A.: Cell. 99, 1) is 3',5' exonuclease having a sequence homologous to RNaseD. Moreover, the nematode rde-2 (Tabara, H., Sarkissian, M., Kelly, W. G., Fleenor, J., Grishok, A., Timmons, L., Fire, A., Mello, C. C.: Cell. 99, 123-132, 1999) is also associated with this stage.

Furthermore, with regard to RNA-dependent RNA polymerases, homologues have been reported, such as qde-1 of fungi, ego-1 of nematodes, and sgs2/sde1 of Arabidopsis (Matzke, M. A., MatzkeA., J. M., Pruss, G., and Vance, V., Curr. Opin. Genet. Dev. 11, 221, 2001). However, the precise role thereof has not yet been clarified.

From the results of analysis of mutant strains, it has also been reported that enzymes classified into helicase are involved in RNAi. Examples may include: qde-3 (Cogoni, C., Macino, G., Science, 286, 2342-2344, 1999) of fungi; smg-2 (Domeier, M. E., Morse, D. P., Knight, S. W., Porteiko, M., Bass, B. L., Mango, S. E., Science, 289, 1928-1931, 2000) of nematodes; mut-6 (Wu-Scharf, D., Jeong, B., Zhang, C., Cerutti, H., Science, 290, 1159-1162, 2000) of Chlamydomonas; and sde-3 (Dalmay, T., Horsefield, R, Braunstein, T. H., Baulcombe, D. C., EMBO J., 20, 2069- 2078) of Arabidopsis.

The presence of other pathways for processing dsRNA is also suggested. HC-Pro (helper component proteinase), which is a plant virus-inhibiting factor of PTGS, inhibits the accumulation of siRNA (small interfering RNA) required for PTGS (Mallory M. F., Matzke, A., Matzke, M., Curr. Biol., 11, 1119, 2001; Llave, C., Kasschau, K. D., Carrington, C., Proc. Natul. Acad. Sci. U.S.A. 97, 13401, 2001). As mentioned above, in the case of a factor reducing RNAi sensitivity, it is considered that the RNAi sensitivity can be increased by repressing the expression of the factor by genetic manipulation, .

Moreover, RNA-directed DNA methylase needs dsRNA, which is similar with RNA that guides the target mRNA in RNAi, and is decomposed in low molecular RNA (Mette, M. F., Aufsatz, W., Van der Winden, J., Matzke, M. A., Matzke, A. J. M., EMBO J. 19, 5194, 2000).

### (2) Introduction of RNAi-associated gene into ES cells

The ES cells (embryonic stem cells) of the present invention can be produced, for example, by the following procedures.

Namely, an RNAi-associated gene is obtained, and an expression vector containing the gene in such a way that the gene can be expressed in ES cells is then constructed. Subsequently, the expression vector is introduced into ES cells which are undifferentiated cells having embryological totipotency. After the introduced RNAi-associated gene is expressed, ES cells having enhanced RNAi effect are isolated.

The type of the expression vector containing an RNAi-associated gene is not particularly limited as long as it can cause the RNAi-associated gene to be expressed in ES cells. The construction of such an expression vector can be appropriately carried out by a person skilled in the art. In general, the RNAi-associated gene is located downstream of a promoter sequence capable of operating in mammals. By adopting such a structure, the RNAi-associated gene can be expressed in mammalian cells. In addition, the expression vector may comprise an enhancer sequence upstream of the promoter sequence. Moreover, the expression vector may also comprise an insulator sequence or a part thereof. For example, preferred examples can be selected from those described later in the specification relative to a recombinant gene comprising the inverted repeat sequence of a target gene, and they can be used as a promoter sequence, enhancer sequence, or insulator sequence to construct the expression vector.

The expression vector containing the RNAi-associated gene can be introduced into ES cells according to conventional methods such as electroporation. ES cells are undifferentiated cells which are established from the inner cell mass of an animal blastocyst and have embryological totipotency. If ES cells are introduced into the early embryo, the cells have properties of being blended together with the cells of the host embryo and continuing development. The type of ES cells used in the present invention is not particularly limited. Further, animals from which the ES cells are derived are not particularly limited either. However, animals used herein are preferably mammals, and either humans or non-human mammals such as mouse, rat, hamster, guinea pig, rabbit, canine, feline, horse, bovine, sheep, swine, goat, and monkey may be used.

It is preferable that ES cells into which an expression vector containing an RNAi-associated gene is to be introduced are cultured and maintained in an undifferentiated state so that they do not lose their potency for differentiating into germ cells. For that purpose, preferably, appropriate nutritive cells are added as feeder cells to a medium, human LIF (leukemia inhibitory factor) is also added thereto, and fetal bovine serum, nucleoside, nonessential amino acid, 2-mercaptoethanol, and others are further added thereto for culture. Preferable examples of nutritive cells may include STO cells and mouse embryo fibroblast cells.

In order to introduce an expression vector into ES cells by electroporation, it is preferable that the cells are suspended in a buffer solution such that the concentration of the cells is kept constant, that the suspension is treated with appropriate restriction enzymes and an expression vector containing a linearized RNAi-associated gene is added thereto, and that electroporation is then carried out thereon under appropriate conditions using an appropriate electroporation apparatus. The pH of a buffer solution used herein is preferably adjusted to 7.0 using PBS or the like.

After the expression vector containing the RNAi-associated gene is introduced into ES cells, the cells are cultured in the above medium for approximately 24 hours. Thereafter, the medium is exchanged with a medium containing a drug (for example, an antibiotic, etc.) to be used in the subsequent selection. Such a drug-containing medium is preferably exchanged with a fresh medium every day, and the culture is continued for approximately 1 week. Thereafter, drug-resistant clones obtained by introduction of the expression vector are picked up, and clones having desired properties are then isolated. It can be confirmed in an appropriate RNAi evaluation system whether or not the isolated ES cells actually have enhanced RNAi effect.

### (3) RNAi evaluation system using recombinant gene comprising inverted repeat sequence

RNAi can be evaluated by using a recombinant gene containing the inverted repeat sequence of a target gene capable of being expressed in mammalian cells. This is to say, a recombinant vector having such a structure is introduced into mammalian cells, so that the inverted repeat sequence of a target gene can be expressed in the cells. Thus, the expression of the target gene can be repressed by RNAi (RNA interference) effects. Such an RNA evaluation system is constructed using ES cells. Thereafter, an expression vector containing an RNAi-associated gene is introduced into the ES cells, so as to evaluate whether or not the degree of repression of the target gene by RNAi effect is enhanced.

The term "inverted repeat sequence" is used to mean a sequence wherein a target sequence is aligned in paralleled with its inverted sequence via a suitable sequence. More specifically, when a target gene has the following double strand consisting of n number of nucleotides:
5'-X₁X₂ ...... Xₙ₋₁Xₙ-3'
3'-Y₁Y₂ ...... Yₙ₋₁Yₙ-5' its inverted sequence has the following sequence:
5'-YₙXₙ₋₁ ...... Y₂Y₁-3'
3'-XₙXₙ₋₁ ...... X₂X₁-5'
wherein when a nucleotide represented by X and a nucleotide represented by Y have the same numerical subscript, these nucleotides are complementary to each other.

The inverted repeat sequence is a sequence wherein the two above types of sequences are aligned in parallel via a suitable sequence. It is considered that there are two cases related to such an inverted repeat sequence: a case where the sequence of a target gene is located upstream of the inverted sequence; and a case where the inverted sequence is located upstream of the sequence of a target gene. The inverted repeat sequences of both the above cases may be used in the present invention, but preferably, the inverted sequence is located upstream of the sequence of a target gene.

A sequence existing between the target gene sequence and the inverted sequence thereof is a region which forms a hairpin loop when it is transcribed into RNA. The length of this region is not particularly limited, as long as it can form a hairpin loop, but it is generally between 0 bp and 700 bp, preferably approximately between 0 bp and 300 bp, and more preferably approximately between 0 bp and 100 bp. Restriction sites may also exist in this sequence.

Any given gene can be used as a target gene used in the present invention. When a transgenic animal is produced using a recombinant gene and gene knockout is intended to be performed thereon by RNAi, the target gene is a gene whose expression is intended to be repressed (a gene whose knockout is intended). Such target genes include genes that have been cloned although their functions remain unknown.

Otherwise, the target gene may be a gene of foreign reporter protein or mutant protein thereof. When such a gene of foreign reporter protein or mutant protein thereof is used as a target gene, RNAi effect can be easily detected and evaluated by a transgenic technique using a recombinant gene.

Examples of such a foreign reporter protein may include an enhanced green fluorescent protein, a green fluorescent protein, aequorin, chloramphenicol acetyltransferase, β-galactosidase, luciferase, and β-glucuronidase.

A mutant protein of such a foreign reporter protein is a protein having substitution, deletion, addition and/or insertion of one to several amino acids (for example 1 to 20, preferably 1 to 10, and more preferably 1 to 5 amino acids) relative to the amino acid sequence of the above-described wild-type reporter protein, and preferably such a mutant protein has a function equivalent to or greater than those of the wild-type reporter protein.

Specific examples of gene of a mutant protein of a reporter protein may include a gene that lacks a part of the nucleotide sequence of a reporter protein gene, a gene wherein the nucleotide sequence of a reporter protein gene is substituted by another nucleotide sequence, and a gene wherein another nucleotide sequence is inserted into a part of a reporter gene. The number of nucleotides that are deleted, substituted or added is not particularly limited, but the number is generally between 1 and 60, preferably between 1 and 30, and more preferably between 1 and 10. In addition, it is desired that these mutant genes maintain their functions as reporter genes.

The gene of the mutant protein can be produced by any given method previously known to a person skilled in the art, such as chemical synthesis, genetic engineering, or mutagenesis. More specifically, a drug acting as a mutagen to DNA encoding a native reporter protein may come into contact with the DNA, ultraviolet rays may be applied, or genetic engineering methods such as PCR method may be used, whereby a gene encoding a mutant protein can be obtained. The site-directed mutagenesis which is one of genetic engineering methods, is particularly useful because it enables introduction of a specific mutation into a specific site. The site-directed mutagenesis can be carried out according to the methods described in Molecular Cloning: A laboratory Mannual, 2^{nd} ED., Cold Spring Harbor Laboratory, Cold Spring Harbor, NY., 1989, Current Protocols in Molecular Biology, Supplement 1 to 38, John Wiley & Sons (1987-1997), etc.

In the recombinant gene that can be used in the present invention, the inverted repeat sequence of a target gene is located downstream of a promoter sequence capable of operating in mammals. By adopting such a structure, the inverted repeat sequence of a target gene can be expressed in mammalian cells. This is to say, in the recombinant gene that can be used in the present invention, the inverted repeat sequence of a target gene is located in such a way that it is located under the control of the above promoter.

The promoter sequence which is used in the present invention is not particularly limited, as long as it can operate in mammals.

Examples of a promoter capable of operating in non-human animals may include gene promoters derived from viruses (for example, Cytomegalovirus, Moloney leukemia virus, JC virus, mammary tumor virus, etc.), and promoters derived from various mammals (for example, human, rabbit, canine, feline, guinea pig, hamster, rat, mouse, etc.). Specific examples of promoters derived from various mammals may include promoters from albumin, endothelin, osteocalcin, muscle creatine kinase, collagen types I and II, a cyclic AMP-dependent protein kinase β subunit (The Journal of Biological Chemistry, Vol. 271, No. 3, pp. 1638-1644, 1996), an atrial natriuretic factor, dopamine β-hydroxylase, a neurofilament light chain (The Journal of Biological Chemistry, Vol. 270, No. 43, pp. 25739-25745, 1995; and of the same publication, Vol. 272, No. 40, pp. 25112-25120, 1997), metallothionein, a metalloproteinase-1 tissue inhibitor, smooth muscle α-actin, a polypeptide chain elongation factor-1α (EF-1α), β-actin, α- and β-myosin heavy chains, myosin light chains 1 and 2, a myelin basic protein, serum amyloid P component, and renin.

Other than the above-described examples, for example, a promoter described in such a publication as Molecular Medicine, extra edition, Manual Disease-Model Mice, edited by Kenichi Yamamura, Motoya Katsuki, and Shinichi Aizawa, Nakayama Shoten Co., Ltd., can also be used.

A human EF1α promoter used in the examples of the present specification is an example of a preferred promoter used in the present invention, but the following promoters may also be used.
(1) β-actin promoter
   In general, a β-actin promoter is used in combination with a CMV enhancer. Examples may include pCAGGS, a chicken beta-actin promoter and a cytomegalo virus enhancer, and beta-actin intron and bovine globin poly-adenylation signal. See H. Niwa, K. Yamanami, J. Miyazaki, Gene, 108, (1991) 193-199 as a reference.
(2) CMV promoter
   In general, a CMV promoter is used in combination with a CMV enhancer. See Janet A. Sawicki et al., Experimental Cell Research 244, 367-369 (1998) as a reference.
(3) Metallothionein promoter
   See Establishment of Transgenic Mice Carrying Human Fetus-Specific CYP3A7, Yong Li et al, Archives of Biochemistry and Biophysics, Vol. 329, No. 2, 235-240, 1996 as a reference.
(4) Apolipoprotein E promoter
   Apolipoprotein E promoter is a promoter for the purpose of the expression in fetal liver. See Simonet et al., 1993, J. Biol. Chem., 268, 8221-8229 as a reference.
(5) Promoter as a gene intended to be introduced

This case includes introduction of the genome itself to produce a transgenic mouse. See Okamoto M. et al., J. Exp. Med., 175, 71 (1992) as a reference.

The recombinant gene that can be used in the present invention may comprise an enhancer sequence upstream of a promoter sequence. The above CMV enhancer is an example of an enhancer sequences used herein.

The recombinant gene that can be used in the present invention may comprise an insulator sequence or a part thereof. The term "insulator sequence" is used to mean a gene sequence that prevents the repression of gene expression caused by the "position effect" in transgenic animals. The insulator sequence is expected to act as a barrier against the influence of neighboring cis-elements.

The location of such an insulator sequence or a part thereof is not particularly limited. In terms of its effects, however, it is preferably located on the 5'-side (upstream) of an introduced gene (that is, the inverted repeat sequence of a target gene). Most preferably, an insulator sequence or a part thereof is located upstream of a promoter sequence (or when an enhancer sequence exists, it is located upstream of the enhancer sequence).

Other than a chicken β-globin-derived insulator sequence described in the examples of the present specification, examples of an insulator sequence that can be used in the present invention may include the following sequences, but are not limited thereto.
(1) Drosophila scs and scs' sequence
   Rebecca Kellum and Paul Schedl, Cell, Vol. 64, 941-950, March 8, 1991
(2) Drosophila gypsy transposon insulator sequence
   Holdrige, C., and D. Dorsett, 1991 Mol. Cell. Biol. 11: 1894-1900
(3) Sea urchin arylsulfatase insulator sequence
   Koji Akasaka et. al., Cellular and Molecular Biology 45 ( 5 ), 555-565, 1999
(4) Human T cell receptor α/δ locus BEAD element
   Zhong, X. P., and M. S. Krangel, 1997, Proc. Natul. Acad. Sci. U.S.A.
(5) Human apolipoprotein B-100 (apoB) matrix attachment site
   Namciu et al, 1998, Mol. Cell. Biol. 18: 2382-2391

The recombinant gene that can be used in the present invention may comprise a poly(A) addition signal sequence downstream of the inverted repeat sequence of a target gene. By inserting the poly(A) addition signal sequence, transcription of messenger RNA of interest can be terminated.

A specific example of such a poly(A) addition signal sequence may include an SV40 poly(A) addition signal, but examples are not limited thereto.

Specific examples of cells produced by the above-described method may include a clone d2 GFP-r6#5 and a clone GFP-r19#4. These clones d2 GFP-r6#5 and GFP-r19#4 were deposited with the National Institute of Advanced Industrial Science and Technology, an Independent Administrative Institution under the Ministry of Economy, Trade and Industry, at the AIST (Tsukuba Central 6, Higashi 1-1-1, Tsukuba, Ibaraki, Japan) under accession Nos. FERM P-18574 and FERM P-18575, respectively, on October 31, 2001.

The clone d2 GFP-r6#5 deposited under accession No. FERM P-18574 was transferred to an international deposition on October 16, 2002, and received accession No. FERM BP-8208. The clone GFP-r19#4 deposited under accession Nos. FERM P-18575 was then transferred to an international deposition on October 16, 2002, and received accession No. FERM BP-8209. These cells have totipotency as mouse embryonic stem cells. The cells express an EGFP protein and emit green fluorescence, and the cells express a nematode rde-1 gene and exhibit high sensitivity to RNAi.

### (4) Non-human mammal having enhanced RNAi effect

In the present invention, ES cells having enhanced RNAi effect obtained as describe above are injected into an animal embryo, and the embryo is then implanted into the uterus of a host, so as to obtain a germ-line chimeric animal. When this chimeric animal is mated with a normal animal, a heterozygous animal (+/-) can be obtained. When two heterozygous animals are mated, a homozygous animal (+/+) can be obtained. By this method, a mammal having enhanced RNAi effect can be obtained.

The type of a mammal is not particularly limited as long as it is a non-human mammal. Specific examples thereof may include mouse, rat, hamster, guinea pig, rabbit, canine, feline, horse, bovine, sheep, swine, goat, and monkey.

A mammal having enhanced RNAi effect can be produced as follows. First, heterozygote ES cells are injected into animal embryos, and the embryos are then cultured *in vitro.* Thereafter, the cultured embryos are implanted into the uterus of a host. The animal embryos used herein are preferably in a state of development from that of 8-cell-stage embryos to that of blastocysts. Generally, 10 to 15 ES cells are injected into animal embryos. Thus, the embryos into which the ES cells are injected are cultured *in vitro* and then implanted into the uterus of a host. Chimeric animals are selected from progenies, which were born from a host impregnated by the above method. Chimeric animals with a high contribution ratio of chimerism are highly likely to be germ-line chimeric animals. By mating a chimeric animal with a normal animal, it is possible to determine whether or not it is a germ-line chimeric animal. Thus, a heterozygous animal that is a germ-line chimeric animal can be obtained, and a homozygous animal can be obtained by mating the two heterozygous animals. The thus obtained animal is characterized in that it has enhanced RNAi effect.

The above animal has genes inherited from a transgenic mammal wherein RNAi-associated genes are incorporated into chromosomes of all the cells thereof, including germinal cells. Progenies of the animal also have the same above genes. A homozygous animal wherein the introduced genes are incorporated into both the homologous chromosomes is obtained, and the female homozygous animal is mated with the male homozygous animal, so that all the progenies are able to stably possess the above gene. Moreover, after confirming that the animals have the above gene, they can be subjected to breeding and passage under a common breeding environment.

All of the contents as disclosed in the specification of Japanese Patent Application No. 2001-348705, which is a priority document of the present application, are incorporated herein by reference in their entirety.

The present invention will be described in detail in the following examples. However, the present invention is not limited by the examples.

### Examples

### Example 1: Establishment of d2EGFP ES cells and construction of RNAi effect measurement system

When EGFP is used as a target gene, since the EGFP has a half-life of 24 hours or longer, even if the translation level of the EGFP is decreased, there is a possibility that RNAi effects might disappear, while masked by the carry-in EGFP that had been translated before introduction of EGFP dsRNA.

Hence, by using EGFP (d2EGFP, pd2EGFP-1 manufactured by Clontech) which was modified in such a way that the EGFP protein had a half-life of 2 hours, a new ES cell transfectant was established. A d2EGFP expression vector used in the above establishment was prepared by inserting, in a positive direction, a BamHI fragment containing the 5'-insulator sequence, CMV enhancer sequence and EF-1α sequence of pUC19 5',3' INS240CE EGFP (which is described in Japanese Patent Application No. 2001-046089; the construction method will be described later) into the BamHI site of a pd2EGFP-1multicloning site manufactured by Clontech. The obtained expression vector was named as pd2EGFP 5' INS240 CE.

pUC19 5',3' INS240CE EGFP was constructed as follows. The *Xho*I*-Afl*II fragment of pCE-EGFP-1 (publication: Takada, T. et al, Selective production of transgenic mice using green fluorescent protein as a marker. Nature Biotech. 15: 458-461, 1997) was inserted by two steps and ligated to the *Xho*I*-Afl*II site of pUC19 5',3' INS240 (which was obtained by chemically synthesizing 10 fragments of a chicken β-globin-derived insulator sequence gene, ligating these fragments with DNA ligase, and inserting the obtained a fragment of 240 base pairs into the multicloning site of a pUC19 vector), and *Escherichia coli* JM109 was transformed therewith, so as to obtain a plasmid pUC19 5',3' INS240 CE EGFP.

The thus prepared vector pd2EGFP 5' INS240 CE was cleaved with restriction enzymes *EcoRI* and *Bsa*I*,* and agarose gel electrophoresis was performed to separate it from the sequence derived from *Escherichia coli.* CCE ES cells (Tetracarcinomas and Embryonic Stem Cells: A practical Approach, Robertson EJ. IRL Press: London, pp.71-112, 1987) were transfected with the obtained gene fragment by electroporation (an electro cell manipulator ECM600 manufactured by BTX). The transfected cells were selected in the presence of 250 µg/ml neomycin, and the obtained colonies were observed with a fluorescence microscope, whereby it was confirmed that the EGFP fluorescence was positive. Thereafter, the cell was defined as d2EGFP ES cell line. The ES cells were cultured by the above-described Robertson EJ's method.

Detection of RNAi effect was carried out by the following method. The ES cells grown on feeder cells were peeled off with trypsin-EDTA, and the cells were inoculated on a gelatin plate at a concentration of 2.1 x 10E4 cells/cm², followed by culture. 24 hours later, the cells were transfected with a plasmid pUC19 5' INS240 EGFP IR having an EGFP dsRNA expression gene (inverted repeat sequence gene), using a gene transfection reagent Lipofectamine 2000. As a control, the same transfection procedure was carried out using a plasmid having an HPRT (Hypoxanthine phosphoribosyltransferase) dsRNA expression gene (inverted repeat sequence gene). 24 hours later, cells were recovered using trypsin-EDTA, and the recovered cells were divided into each single cell. Thereafter, fluorescence of the cell was analyzed with FACScan (BD).

As the plasmid pUC19 5' INS240 EGFP IR having an EGFP dsRNA expression gene (inverted repeat sequence gene), a plasmid described in Japanese Patent Application No. 2001-046089 was used. This is to say, the *kpn*I*-Xho*I fragment of Litmus28 EGFP was inserted into the *Kpn*I*-Sal*I cleavage site of the above prepared pUC19 5',3' INS240 CE EGFP, and they were ligated to each other. Thereafter, *Escherichia coli* SURE2 strain (Stratagene) was transformed therewith, so as to obtain a plasmid pUC19 5'INS240 CE EGFP IR having an inverted repeat sequence

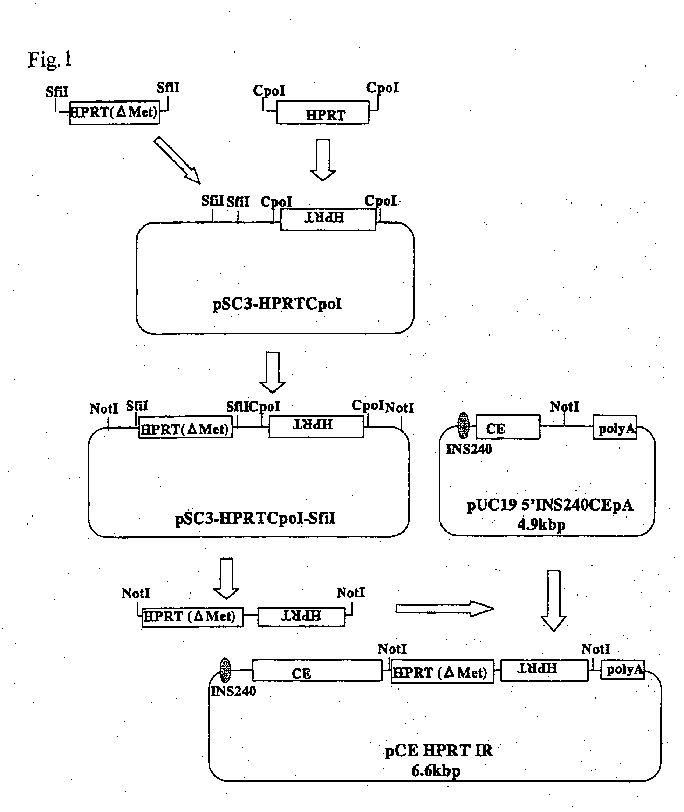

The HPRT dsRNA expression gene used as a control was constructed by the following method (Figure 1).

With regard to a hypoxanthine phosphoribosyltransferase gene, ATCC No. 37424 was purchased, a fragment was cut off with restriction enzymes *Age*I and *Bal*I*,* and it was then cloned into a Litmus28 vector. Using this vector as a template, PCR was carried out using primers containing a restriction site *Cpo*I or *Shi*I (SEQ ID NOS: 1 and 2, or 3 and 4). The PCR condition was 93°C, 3 minutes, (93°C, 30 seconds, 55°C, 30 seconds, and 72°C, 1 minute) x 25 cycles, and 72°C, 10 minutes. The obtained DNA fragment was treated with *Cpo*I or *Sfi*I. Agarose gel electrophoresis was carried out thereon, and then DNA fragments were recovered. On the other hand, a pSC3 vector (FEBS Letters 479, 79-82, 2000) provided from Mr. Zenno, Graduate School of Science, the University of Tokyo, was treated with *Cpo*I*.* Agarose gel electrophoresis was carried out thereon, and then DNA fragments were recovered. The HPRT DNA fragment-*Cpo*I was ligated to the pSC3 vector-*Cpo*I, and *Escherichia coli* JM109 strain was transformed therewith, so as to obtain a plasmid pSC3-HPRTC*po*I. Subsequently, the pSC3-HPRT*Cpo*I was treated with *Sfi*I, and then electrophoresed in an agarose gel, and DNA fragments were recovered. The HPRT DNA fragment-*Sfi*I was ligated to pSC3-HPRT*Cpo*I-*Sfi*I, and *Escherichia coli* SURE2 strain (Stratagene) was transformed therewith, so as to obtain a plasmid pSC3-HPRT*Cpo*I-S*fi*I having an inverted repeat sequence.

Moreover, the plasmid pSC3-HPRT*Cpo*I-*Sfi*I was treated with *Not*I*,* and then electrophoresed in an agarose gel, and the DNA fragment having a size of approximately 1.7 kbp was recovered from the gel. Thereafter, pUC19 5'INS240 CE/EGFP IR (as stated above, described in Japanese Patent Application No. 2001-046089) was treated with *Not*I*,* and EGFP IR fragments were then cut off, followed by self-ligation, so as to prepare a pUC19 5'INS240 CEpA vector. The pUC19 5'INS240 CEpA vector was treated with *Not*I*,* and thereafter, a dephosphorylation treatment was carried out with BAP to prevent self-ligation. Thereafter, phenol extraction, chloroform extraction, and ethanol precipitation was carried out to remove BAP.

pSC3-HPRT*Cpo*I-*Sfi*I-*Not*I was ligated to a pUC19 5'INS240 CEpA vector-*Not*I site, and *Escherichia coli* SURE2 strain (Stratagene) was transformed therewith, so as to obtain a plasmid pCE HPRT IR having an inverted repeat sequence.

### Example 2: Construction of expression vector cassette having RNAi-associated gene

In order to express various RNAi-associated genes in mammalian cells, a cassette vector having a puromycin-resistant gene which is driven by a CMV enhancer and an EF-1α promoter was produced.

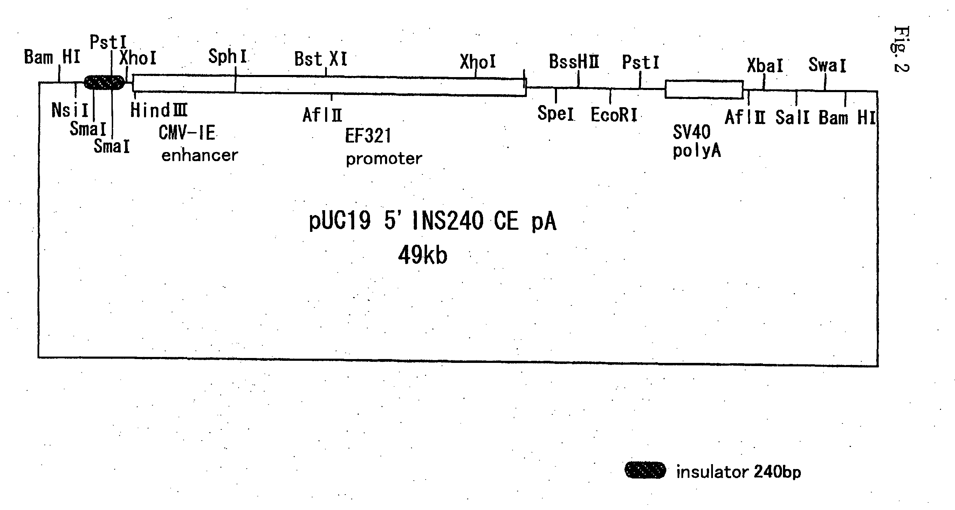

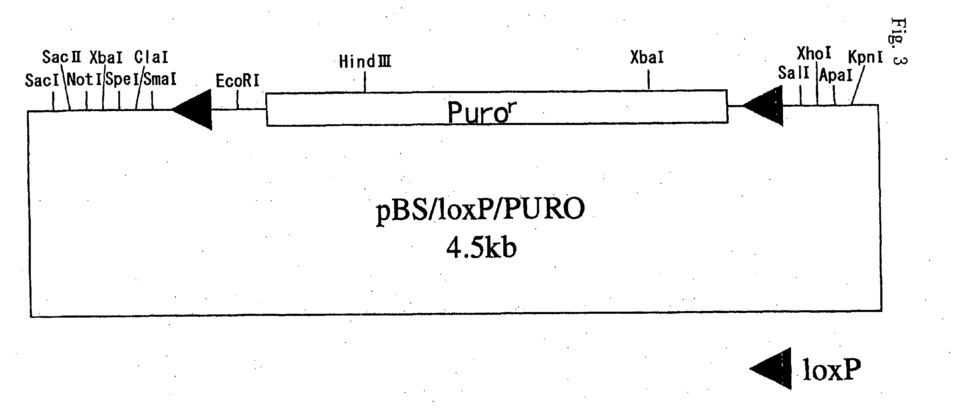

That is to say, a *Spe*I-*Bss*HII-*Eco*RI linker (SEQ ID NOS: 5 and 6) was inserted into the *Eco*RI site of the pUC19 5'INS240 CEpA vector, and a *Xba*I-*Sa*II-*Swa*I linker (SEQ ID NOS: 7 and 8) was inserted into the *Swa*I site thereof, so as to produce a pUC19 5'INS240 CEpA XSS vector (Figure 2).
(A) Production of mut-7 expression gene
   (1) Cloning of mut-7 gene
      Cloning of a mut-7 gene (GENEBANK SEQUENCE NO: ZK1098) was carried out using a C. elegans cDNA library (Genes to Cells 3,189-202 (1998) for expression in yeasts, which had been provided from Mr. Sugimoto, Department of Science, the University of Tokyo). *Escherichia coli* XL-10 Gold (Stratagene) was transformed with this cDNA library, and colony hybridization was carried out. A region on the 5'-side (SEQ ID NOS: 9 and 10) of mut-7 cDNA that had been amplified by PCR using the cDNA library as a template was used as a probe. As a result, two mut-7 clones were obtained. A cDNA region was cleaved therefrom with *Bss*HII*lBam*HI*,* and the region was inserted into the *Bss*HII*lBam*HI site of Litmus28 (New England Biolabs), so as to produce L28/mut-7. Both the clones were recombined using *Bg*lII/*Pst*I*,* so as to obtain a cDNA clone containing a full-length ORF having no amino acid mutation. Thereafter, the *Spe*I*lEco*RI cDNA fragment of the L28/mut-7 was inserted into the *Nhe*I*lEco*RI site of Litmus 38 (New England Biolabs), so as to obtain L38/mut-7.
   (2) Introduction of mut-7 gene into vector
      The *Mlu*I*lEco*RI fragment of the L38/mut-7 was inserted into the *Bss*HII*lEco*RI site of the pUC19 5' INS240 CEpA XSS vector, so as to obtain pUC19 5' INS240 CE/mut-7. *Escherichia coli* SCS110 (Stratagene) was transformed with this plasmid, so as to obtain a demethylated plasmid.
   (3) Construction of PBS/LoxP/Puro
      The *Bam*HI site of PloxP (Kitamoto T. et al., Biochem. And Biophys. Res. Commun. 222, 742-747 (1996)) was treated with restriction enzymes and then blunted, followed by self-ligation to crush it. Thereafter, a *Bgl*II linker (SEQ ID NO: 11) was inserted into the *Hind*III site thereof, so that the *Hin*dIII site was converted into a *Bgl*II site. The *Pvu*II/*Bam*HI fragment of pPUR (Clontech) was inserted into the *Eco*RV/*Bgl*II site of the thus obtained vector, so as to obtain pBS/loxP/Puro (Figure 3).
   (4) Construction of pUC19 5' INS240 CE/mut-7/loxP/Puro

   The *Spe*I/*Xho*I fragment of the pBS/loxP/Puro was inserted into the *Xba*I/*Sal*I site of the pUC19 5' INS240 CE/mut-7, so as to obtain pUC19 5' INS240 CE/mut-7/loxP/Puro. This plasmid was treated with *Bam*HI*,* so as to obtain a gene fragment having a size of approximately 6.5 kb.
(B) Production of rde-1 expression gene
   (1) Obtainment of rde-1 gene
      The cDNA clone of rde-1 was provided from Dr. Ohara of the National Institute of Genetics based on Accession No. AF180730 of GENEBANK. pBluescriptSK-/rde-1 was obtained from the phage clone by in vivo excision.
   (2) Construction of vector
      *A Bss*HII*-Not*I*-Eco*RV*-Nhe*I*-Kpn*I linker (SEQ ID NOS: 12 and 13) was inserted into the *Bss*HII site of the pUC19 5' INS240 CEpA XSS, a *Swa*I*-Asc*I*-Pac*I linker (SEQ ID NOS: 14 and 15) was inserted into the *Swa*I site thereof, and an *Nsi*I*-Asc*I*-Pac*I linker (SEQ ID NOS: 16 and 17) was inserted into the *Nsi*I site thereof. Moreover, the *Spe*I*lXho*I fragment of the pBS/loxP/Puro was inserted into the *Xba*I/*Sal*I site thereof, so as to obtain pUC19 5' INS240 CE/BNENK/loxP/Puro (Figure 3).
   (3) Construction of pUC19 5' INS240 CE/rde-1/loxP/Puro

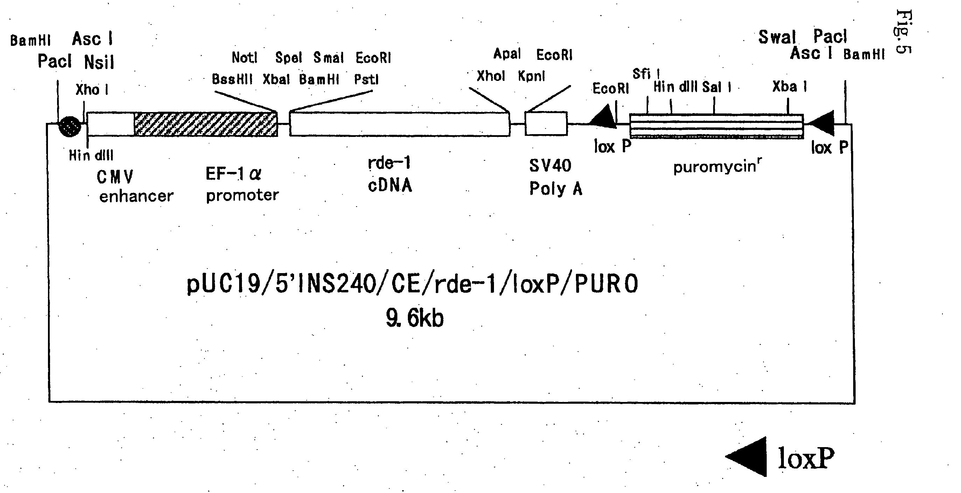

The *Kpn*I*lNot*I fragment of pBluescriptSK-/rde-1 was inserted into the *Kpn*I/*Not*I site of the pUC19 5' INS240 CE/BNENK/loxP/Puro, so as to obtain pUC19 5' INS240 CE/rde-1/loxP/Puro (Figure 5). This plasmid was treated with *Pac*I*,* so as to obtain a gene fragment having a size of approximately 7.0 kb.

### Example 3: Establishment of mut-7 expression ES cell line

A purified mut-7 expression gene was introduced into the d2EGFP expression ES cell line by electroporation. Thereafter, transfected cells were selected in the presence of 600 ng/ml puromycin, thereby obtaining a cell line. In addition, DNA was prepared from the cells, and the presence of a mut-7 gene was detected by Southern hybridization. As a result of the Southern hybridization, a clone containing a band showing a predicted migration was defined as a positive clone. By the same method, a mut-7 expression gene was introduced into an ES cell line (Takada, T., Yoshida, K., Nakamura, K., Nakao, K., Tujimoto, G., Katsuki, M., Sugano, S., Expression of Green Fluorescent Protein in Transgenic Mice. Methods in Enzymology 302, 233-250, 1999), which drives a GFP mut-1 variant having a half-life of 24 hours or longer and comprising two amino acid substitutions (Phe-64 being substituted by Leu and Ser-65 being substituted by Thr) with an EF-1α promoter, so as to obtain positive clones.

### Example 4: Establishment of rde-1 expression ES cell line

A purified rde-1 expression gene was introduced into the d2EGFP expression ES cells by electroporation. Thereafter, the transfected cells were selected in terms of drug resistance to puromycin, so as to obtain a cell line. Thereafter, DNA was prepared from the cells, and the presence of the rde-1 gene was detected by Southern hybridization. As a result of the Southern hybridization, a clone of a band showing a predicted migration was defined as a positive clone. By the same method, a rde-1 expression gene was introduced into the ES cell line (Takada, T., Yoshida, K., Nakamura, K., Nakao, K., Tujimoto, G., Katsuki, M., Sugano, S., Expression of Green Fluorescent Protein in Transgenic Mice. Methods in Enzymology 302, 233-250, 1999), so as to obtain positive clones.

Among the obtained rde-1 expression ES cells, clones d2 GFP-r6#5 and GFP-r19#4 were deposited with the National Institute of Advanced Industrial Science and Technology, an Independent Administrative Institution under the Ministry of Economy, Trade and Industry, at the AIST (Tsukuba Central 6, Higashi 1-1-1, Tsukuba, Ibaraki, Japan) under accession Nos. FERM P-18574 and FERM P-18575, respectively, on October 31, 2001.

The clone d2 GFP-r6#5 deposited under accession No. FERM P-18574 was transferred to an international deposition on October 16, 2002, and received accession Nos FERM BP-8208. The clone GFP-r19#4 deposited under accession No. FERM P-18575 was transferred to an international deposition on October 16, 2002, and received accession No. FERM BP-8209.

### Example 5: Analysis of RNAi effects of RNAi-associated gene-expressing ES cell line

Each ES cells grown on feeder cells were peeled off with trypsin-EDTA, and the cells were inoculated on a gelatin-coated plate at a concentration of 2.1 x 10E4 cells/cm², followed by culture. 24 hours later, the cells were transfected with a plasmid pUC19 5' INS240 EGFP IR having an EGFP dsRNA expression gene (inverted repeat sequence gene), using a gene transfection reagent Lipofectamine 2000. As a control, the same transfection procedure was carried out using a plasmid having an HPRT (Hypoxanthine phosphoribosyltransferase) dsRNA expression gene (inverted repeat sequence gene). 24 hours later, cells were recovered using trypsin-EDTA, and the recovered cells were divided into each single cell. Thereafter, fluorescence of the cell was analyzed with FACScan (BD).

As a result, in the case of the clone d2 GFP-r6#5, it was found that the number of cells with reduced fluorescence was 10% larger than the number of control cells (Figure 6).

Moreover, in the case of the clone GFP-r19#4 that is a cell line obtained by introducing the rde-1 gene into the EGFP ES cell line, it was found that the number of cells with reduced fluorescence was 28% larger than the number of control cells in which the gene was not introduced (Figure 7).

### Example 6: Production of chimeric mouse from ES cells

The ES cells grown on feeder cells were peeled off with trypsin-EDTA. Thereafter, the feeder cells were removed by adhesion on a gelatin-coated plate, and the ES cells were suspended in injection medium (DMEM/20% FBS).

The ES cells were injected into a fertilized ovum, a blastcyst embryo, which was obtained by mating C57BL/6 and BDF1, and the embryo was then implanted into the uterus of a pseudopregnant MCH mouse, so as to obtain a chimeric mouse.

### Industrial Applicability

When the functions of a novel gene are analyzed at an individual fetus or laboratory animal level by the present invention, the results can be obtained more quickly than by the conventional knockout methods. Moreover, in the analysis of disease-associated genes or target genes of pharmaceuticals using RNAi effect, these genes can be repressed more reliably than in the conventional use of mice, and it is therefore considered that the present invention greatly contributes to the industry.

## Claims

1. ES cells having enhanced RNAi effect, which are obtained by performing genetic manipulation on ES cells.

2. The ES cells of claim 1 which are obtained by introducing an RNAi-associated gene into ES cells.

3. The ES cells of claim 1 or 2 wherein the RNAi-associated gene is a gene encoding a factor associated with the formation of a sequence-specific intermediate, a gene encoding a factor associated with a stage of repressing a target gene expression, a gene encoding RNA-dependent RNA polymerase, or a gene encoding helicase.

4. The ES cells of claim 2 or 3 wherein the RNAi-associated gene is nematode rde-1 or rde-4 gene, fungus qde-2 gene, Arabidopsis ago-1 gene, Dicer gene or homologous genes thereof, a gene encoding PAZ/Piwi family proteins, nematode mut-7 gene, nematode rde-2 gene, fungus qde-1 gene, nematode ego-1 gene, Arabidopsis sgs2/sdel gene, fungus qde-3 gene, nematode smg-2 gene, Chlamydomonas mut-6 gene, or Arabidopsis sde-3 gene.

5. The ES cells of claim 4 wherein the RNAi-associated gene is nematode rde-1 gene or nematode mut-7 gene.

6. The ES cells of any of claims 1 to 5 which are obtained by introducing into ES cells an expression vector which has an RNAi-associated gene in such a state that the gene can be expressed in host cells.

7. The ES cells of any of claims 1 to 6 which further comprises a recombinant gene comprising an inverted repeat sequence of a target gene that can be expressed in mammalian cells.

8. The ES cells of claim 7 wherein the recombinant gene comprising an inverted repeat sequence comprises the inverted repeat sequence of the target gene downstream of a promoter sequence capable of operating in mammalian cells;

9. The ES cells of claim 7 or 8 wherein the recombinant gene comprising an inverted repeat sequence comprises an enhancer sequence upstream of the promoter sequence.

10. The ES cells of any of claims 7 to 9 wherein the recombinant gene comprising an inverted repeat sequence further comprises an insulator sequence or a part thereof.

11. The ES cells of any of claims 7 to 10 wherein the recombinant gene comprising an inverted repeat sequence comprises a poly(A) addition signal sequence downstream of the inverted repeat sequence of the target gene.

12. The ES cells of any of claims 7 to 11 wherein the target gene is a gene of a foreign reporter protein or a mutant protein thereof.

13. The ES cells of claim 12 wherein the foreign reporter protein is an enhanced green fluorescent protein (EGFP).

14. The ES cells which have Accession No. FERM BP-8208 (transferred from FERM P-18574) or FERM BP-8209 (transferred from FERM P-18575).

15. A non-human mammal derived from the ES cells of any of claims 1 to 14, or progenies thereof, or a part thereof.

16. The non-human mammal wherein the non-human mammal is selected from the group consisting of mouse, rat, hamster, guinea pig, rabbit, canine, feline, horse, bovine, sheep, swine, goat, and monkey; or progenies thereof; or a part thereof.
